# EUROPEAN PATENT APPLICATION

(11) **EP 4 732 843 A1**
(43) Date of publication of application: **29.04.2026**
(21) Application number: 24306775.8
(22) Date of filing: 22.10.2024
(51) Int. Cl.: A61K 36/06, A61K 31/122, A61P 19/10

(54) **LIVE SACCHAROMYCES CEREVISIAE STRAINS FOR THE TREATMENT OF BONE-LOSS DISORDERS**

(71) Applicant: Lesaffre et Compagnie, 75001 Paris (FR)
(72) Inventor: BOSCO, Nabil, 59700 MARCQ-EN-BAROEUL (FR); CHABRAND, Lucie, 59160 CAPINGHEM (FR); KOPER, Johanna, 9890 GAVERE (BE)
(74) Representative: Plasseraud IP

(57) **Abstract**

The present invention pertains to the use of a live *Saccharomyces cerevisiae* strain for the treatment and/or prevention of bone-loss disorders. The present invention further pertains to a combination of a live *Saccharomyces cerevisiae* strain with vitamin K2 for the treatment and/or prevention of bone-loss disorders such as osteoporosis. The present inventors have shown that such a combination allows significantly improving bone health markers while reducing negative markers associated with osteoclastogenic activity.

## Description

### Technical Field

The present invention pertains to the medical field, in particular to the treatment of bone disorders. The present invention particularly relates to the use of live *Saccharomyces cerevisiae* strains for the treatment of bone-loss disorders such as osteoporosis.

### Background Art

Bones provide a rigid framework and are responsible for the mechanical support of the body. They are dynamic tissues that are tightly regulated and under constant remodeling. Bone remodeling is the result of a strict balance between bone resorption (mediated by osteoclasts) and bone formation (mediated by osteoblasts) to maintain an appropriate bone density and mechanical strength. This balance relies on multiple mediators and a large number of coordinated signaling mechanisms which are essential for maintaining a proper bone function.

Unfortunately, under certain pathological conditions, this balance can be deregulated and lead to abnormal bone remodeling and the development of bone-loss disorders.

Osteoporosis is one of the most common bone-loss disorders. It occurs when bone mineral density and bone mass decrease. It is defined as low bone mineral density caused by altered bone microstructure ultimately predisposing patients to low-impact, fragility fractures and ultimately loss of mobility and poor quality of life. According to the International Osteoporosis Foundation, over 32 million individuals, including 25.5 million women and 6.5 million men, aged 50 and above suffered from osteoporosis in Europe in 2019. This represents approximately 5.6% of the total European population aged 50 and above. The cost of osteoporosis in the EU in 2019 was estimated at €56.9 billion. Worldwide, more than 200 million people have osteoporosis causing more than 8.9 million fractures annually. Over 70% of those over age 80 are affected.

The two main mechanisms by which osteoporosis develops are excessive bone resorption by osteoclast cells and/or insufficient formation of new bone tissue by osteocytes/osteoblast cells. Calcium and vitamin D depletion worsen the disease. Hormones play an active role in bone remodeling, and hormone level variations can therefore directly have an impact on bone formation. For example the lack of estrogen resulting from menopause increases bone resorption. In the same vein, the thyroid hormone calcitonin increases bone deposition, and the parathyroid hormone (PTH), secreted by the parathyroid glands in response to low-calcium levels induces higher osteoclastic activity to induce calcium release in blood.

Nutrition and dietetic measures, including food supplementation with Vitamin D and calcium have been proposed in prevention of osteoporosis. Vitamin D helps calcium fixation and phosphorus. Some studies show that a combination of Vitamin D with calcium allows limiting osteoporotic fractures in patients (see e.g. Feng et al. Orthop. Surg. 2021; 13:1262-1268). However, such supplementation strategies remain ineffective in patients who are not depleted in calcium and/or vitamin D.

Probiotics comprising bacteria such as *Lactobacillus* strains have been identified as potentially useful in the prevention of osteoporosis. Recently, a combination of vitamin D and *Lactobacillus reuteri* probiotic showed interesting results in terms of bone health. However, further research needs to be carried out to evaluate whether it has any effect on fracture risk (Rizzoli and Biver. Current osteoporosis reports 18 (2020): 273-284).

Alternative therapeutic strategies involve bisphosphonates administration to reduce osteoclast activity or selective estrogen receptor modulators (SERMs) to increase bone formation. Unfortunately, these treatments may be associated with various side-effects and none of them are fully satisfying (Migliaccio, Silvia, Marina Brama, and Giovanni Spera. Clinical Interventions in Aging 2.1 (2007): 55-64).

Accordingly, there is still a need for the provision of novel therapies that would allow preventing and reducing bone loss during osteoporosis. Such therapies could be useful more generally in the treatment of various bone disorders associated with bone loss.

### Summary of the invention

Using a specific model of the human intestinal microbial ecosystem, the present inventors have shown that it is possible to stimulate various bone-formation markers upon administration of a composition comprising a live *Saccharomyces cerevisiae* strain. The inventors have particularly shown that such a composition allows inducing a significant increase in propionate and butyrate levels. Propionate and butyrate are two short-chain fatty acids that are known to be correlated with bone formation. The results provided herein therefore show that it is possible to improve bone formation upon administration of a composition comprising a live *Saccharomyces cerevisiae* strain, and, accordingly, that such a composition can be useful for the treatment of disorders associated with bone loss.

Accordingly, a first aspect of the present invention pertains to a live *Saccharomyces cerevisiae* strain for use in the treatment and/or prevention of a bone-loss disorder.

The live *Saccharomyces cerevisiae* strain can preferably be obtained from the strain deposited on October 17, 2007, at the Collection Nationale de Cultures de Micro-organismes (CNCM) pursuant to the treaty of Budapest under No. CNCM I-3856.

More surprisingly, the present inventors have shown that the live *Saccharomyces cerevisiae* strain can synergistically interact with vitamin K, in particular vitamin K2, and strongly promotes the production of positive bone markers, including propionate, butyrate, indole-3-ethanol and kynurenic acid, while at the same time reducing negative markers associated with osteoclastogenic activity (such as indole-carboxaldehyde). The present invention therefore also relates to a combination therapy comprising a live *Saccharomyces cerevisiae* strain and vitamin K2 for the treatment of bone-loss disorders.

In this context, the present invention pertains to a kit of parts comprising a live *Saccharomyces cerevisiae* strain and vitamin K2. The invention also relates to the use of said kit of parts in therapy, particularly in the treatment and/or treatment of bone-loss disorders, including osteoporosis.

### Brief Description of Drawings

**Figure 1****:** relative short-chain fatty acids (SCFA) concentration after 48 hours of in vitro human fermentation, normalized for the blank. N=2 donors.
**Figure 2****:** Production of indole-3-ethanol after 48h of in vitro fermentation with Vitamin K2, CNCM I-3856 and the combo. N=2 donors.
**Figure 3****:** Production of kynurenic acid after 48h of in vitro fermentation with Vitamin K2, CNCM I-3856 and the combo. N=2 donors.
**Figure 4****:** Production of indole-carboxaldehyde after 48h of in vitro fermentation with Vitamin K2, CNCM I-3856 and the combo. N=2 donors.
**Figure 5****:** Schematic mode of action of the combo Vitamin K2 and CNCM I-3856.

### Detailed description of the invention

According to a first embodiment, the present invention pertains to a live *Saccharomyces cerevisiae* strain for use in the treatment and/or prevention of a bone-loss disorder in a patient in need thereof.

*Saccharomyces cerevisiae,* also referred to as "brewer's yeast" or "baker's yeast" is a well-known species of yeast which is commonly used in winemaking, brewing and baking processes (see for review Parapouli et al. AIMS microbiology 6.1 (2020): 1). *Saccharomyces cerevisiae* strains all possess a nuclear genomic DNA of 12068 kilobases (kb) organized in 16 chromosomes (Goffeau A et al. Science. 1996;274:563-547). In addition to this nuclear genomic DNA, *Saccharomyces cerevisiae* strains also possess extra chromosomal elements which vary between the various strains including mitochondrial DNA (mtDNA) molecules, double-stranded DNA elements and single- and double-stranded RNA molecules and retroviruses.

In the context of the present invention, the *Saccharomyces cerevisiae* strain used can be isolated/obtained from any known *Saccharomyces cerevisiae.* According to a preferred embodiment, the *Saccharomyces cerevisiae* strain can be obtained from the strain deposited on October 17, 2007, at the Collection Nationale de Cultures de Micro-organismes (CNCM, 25/28 rue du docteur ROUX, F-75724 Paris Cedex 15) pursuant to the treaty of Budapest under No. CNCM I-3856. The strain can e.g. be obtained by isolating a yeast from strain CNCM I-3856 and culturing it. Indeed, as shown in the experimental section below, this specific strain is particularly efficient for inducing a strong increase in the synthesis of positive bone markers such as the short-chain fatty acids propionate and butyrate, as well as indole-3-ethanol, which is correlated with bone formation, and kynurenic acid, which is a negative marker of bone resorption. This strain has been described e.g. in patent applications published under references WO2009/103884 (for the prevention/treatment of gastrointestinal disorders), WO2014/009656 (for preventing/treating vaginal mycoses), WO2020/127136 (for the treatment/prevention of oropharyngeal candidiasis) and WO2020/245057 (for the treatment/prevention of simple and/or recurring cystitis). The *Saccharomyces cerevisiae* strain according to the present invention may be cultured by means of any suitable method known by the skilled person. Methods for culture of yeasts are known in the prior art, and a person skilled in the art knows how to optimize the culture conditions for each strain as a function of its nature. A yeast is obtained by multiplication of a yeast strain in a culture medium, for example as described in the reference book "Yeast Technology", 2nd edition, 1991, G. Reed and T. W. Nagodawithana, published by Van Nostrand Reinhold, ISBN 0-442-31892-8. Thus, for example, on an industrial scale, yeast cells usable in the context of the present invention may be obtained by a method comprising the following steps:
- culturing a yeast strain in a culture medium in several stages, firstly in semi-anaerobiosis, then in aerobiosis (oxygen-rich medium/atmosphere) to obtain multiplication of the starting yeast;
- separating, by centrifugation, the yeast cells obtained; and
- prepare the yeasts in an appropriate form such as a liquid yeast cream containing between 12% and 25% of yeast dry matter.

In the context of the present invention, the strain used is alive, i.e. a live *Saccharomyces cerevisiae* strain. By a "live *Saccharomyces cerevisiae* strain" is meant that the metabolism of the *Saccharomyces cerevisiae* strain is active or reactivatable or capable of multiplying.

The live *Saccharomyces cerevisiae* strain can be in any form suitable for administration in a patient. The strain is advantageously formulated as a probiotic, i.e. formulated for oral administration. The skilled person knows how to formulate live *Saccharomyces cerevisiae* strains for oral administration. The strain can e.g. be in a fresh form such as a cream yeast or compressed yeast, or in a dry form such as a dry yeast or frozen yeast.

Fresh yeasts are characterized by a high water-content compared with dry yeasts. Fresh yeasts encompass cream yeasts and compressed or crumbled yeasts.

Cream yeasts, also known as "liquid yeasts", are aqueous suspensions of yeast cells having a cream-type viscosity. These aqueous suspensions of living yeast cells generally have a solid content of at least 12% by weight, particularly from 12 to 40 % by weight. A cream yeast may have, for example, a solid content of between 12 and 25% by weight, preferably between 14 and 22% by weight. In such a case, the yeast is preferably encapsulated. The encapsulation methods and the different types of capsules are well known to one skilled in the art.

Compressed yeasts comprise yeasts compressed into a compact block and crumbled compressed yeasts. Compressed yeasts in a compact block, also known as "yeast bars", are characterized by a solids content of between 26% and 35%. Crumbled compressed yeasts have a water content of between 21% and 35%.

In the context of the present invention, the live yeast is preferably in the form of a dry yeast. When present in a dry form, the yeast can be in the form of an instantaneous dry or active dry form. Dry forms generally comprise a dry material level above 90%, preferably ranging from about 92%-96%.

Frozen yeasts are characterized by a solid content of between 74% and 80%.

Typically, the daily dosage of live *Saccharomyces cerevisiae* yeast administered to the patient in the context of the present invention is comprised between 10⁷ and 6×10¹⁰ CFU (Colony Forming Unit), preferably between 10⁸ and 2×10¹⁰ CFU.

The present inventors have shown that the use of a live *Saccharomyces cerevisiae* strain alone, without requiring the presence of any other microorganism such as bacteria (including *Lactobacillus* stains), allows improving bone health markers. Therefore, in the context of the present invention, the live *Saccharomyces cerevisiae* strain does not need to be administered in combination with another microorganism. Accordingly, according to a further embodiment, the live *Saccharomyces cerevisiae* is not administered in combination with a microorganism that does not belong to the *Saccharomyces cerevisiae* species. This means that the treatment/prevention of the bone-loss disorder according to the present invention does not comprise the administration of another microorganism than the live *Saccharomyces cerevisiae* strain.

In the context of the present invention, the "bone-loss" disorder refers to a skeletal disorder comprising a decrease in bone density, strength and/or mass. Bone-loss disorders are typically correlated with an imbalance in bone remodeling, in particular with an increased osteoclastogenic activity and a reduced osteoblastogenic activity, thereby leading to bone resorption and/or increased fragility. Bone-loss disorders include osteoporosis, osteopenia, osteoarthritis, Paget's disease, osteomyelitis, osteomalacia, degenerative joint disease, musculoskeletal pathologies, osteophytes, rickets and delayed or non-union fractures.

According to a specific embodiment, the bone-loss disorder treated in the context of the present invention is a low bone-density disorder such as osteoporosis and osteopenia. Low bone-density disorders are well characterized. Bone density is generally measured with dual-energy X-ray absorptiometry (DEXA). It allows providing a score referred to as a T-score. A T-score comprised between +1 and -1 indicates normal bone density, between -1 to -2.5 indicates osteopenia and of -2.5 and below indicates osteoporosis.

In the context of the present invention, the "patient" or "subject" refers to any human or animal suffering from a bone-loss disorder. Typically, the patient is a mammal. The patient can e.g. be a human, a feline such as a cat, a canine such as a dog or an equid such as a horse. Preferably, the patient is human.

According to the present invention the term "preventing" a disease or disorder means avoiding its onset, particularly in individuals at risk of developing it, or delaying its symptoms or repercussions. In this context, it is therefore referred to the preventive or prophylactic treatment of this disorder. In particular, "preventing" consists in administering the strain before the disorder appears.

The term "treating" a disease or disorder means treating said disease/disorder in order to cure it, alleviate its symptoms or repercussions, or at least stabilize it. In this context, it is therefore referred to the curative treatment of this disorder.

In particular, "treating" consists in administering the strain when the disease or disorder has set in.

The terms "reduce the severity of symptoms" of a disease/disorder and "reduce the symptoms" of a disease/disorder are used interchangeably and refer to reducing the abnormal manifestation/perceptible clinical sign(s) caused by said disease/disorder.

As explained above, the present inventors have shown that it is possible to significantly potentiate the beneficial effects of the live *Saccharomyces cerevisiae* strain on bone health by combining it with vitamin K2. Indeed, the experimental data provided below shows that the live *Saccharomyces cerevisiae* strain and vitamin K2 act synergistically for stimulating the expression of positive bone formation markers while at the same time reducing the expression of negative markers associated with bone resorption.

Those skilled in the art will understand that vitamin K and derivatives thereof refer to one or more compounds of Formula 1 and their pharmaceutically or nutritionally acceptable salts: 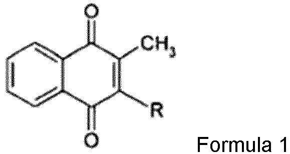 wherein R may be any covalently linked organic group including polyisoprenoid residues, esters, ethers, and thiol adducts.

According to some aspects, the vitamin K may be a vitamin K2, i.e., a menaquinone, selected from the group consisting of short-chain menaquinones (i.e., MK-1 , MK-2, MK-3, and MK- 4), long-chain menaquinones (i.e., MK-5, MK-6, MK-7, MK-8, and MK-9), and combinations thereof. Those skilled in the art will understand that menaquinones are abbreviated MK-n, wherein M represents menaquinone, K represents vitamin K, and n represents the number of isoprenoid side chain residues. According to some aspects, the vitamin K2 may comprise or consist of MK-7 or MK-4.

Sources of vitamin K2 which may be useful according to aspects of the present disclosure include, but are not limited to different forms of vitamin K2 including synthetic MK-4, MK-5, MK-6, MK-7, MK-8,MK-9, MK-10, MK-11, MK12, and MK-13, natto (i.e., food prepared from fermented soybean), fermented foods, dairy products, meat, fish and combinations thereof.

Vitamin K2 promotes bone health and acts in concert with vitamin D to support bone physiology and reduces some side effects of high vitamin D intake like soft tissue (e.g. blood vessel) calcification. It's necessary for blood clotting and promotes the accumulation of calcium in bones and teeth. Vitamin K2 activates osteocalcin, a protein that promotes specific bone and teeth calcium accumulation. There are reports showing poor vitamin K2 status in frail individuals and association with bone fragility. Therefore, a higher intake of vitamin K2 is also recommended for bone health.

Therefore, according to a further embodiment, the present invention pertains to a combination of a live *Saccharomyces cerevisiae* strain with vitamin K2, particularly for the treatment and/or prevention of bone-loss disorders.

In the context of the present invention the live *Saccharomyces cerevisiae* strain and vitamin K2 can be formulated as a 2-in-1 product, comprising both said strain and vitamin K2. Alternatively, the live *Saccharomyces cerevisiae* strain and vitamin K2 can also be administered separately, as two distinct products.

Accordingly, the present invention also pertains to a kit of parts comprising a live *Saccharomyces cerevisiae* strain and vitamin K2.

The term "kit of parts" herein refers to a combined preparation wherein the active ingredients are physically separated for use in a combined therapy by simultaneous administration or sequential administration to the patient.

The present invention particularly pertains to a kit of parts comprising a live *Saccharomyces cerevisiae* strain and vitamin K2, for use in therapy, such as in the treatment and/or prevention of a bone-loss disorder in a patient in need thereof. According to the present invention, the live *Saccharomyces cerevisiae* strain and vitamin K2 are administered to the patient in a separate form, either simultaneously, separately or sequentially in any order to the patient for treating said bone-loss disorder.

According to a specific embodiment, the form of the vitamin K2 used in the context of the present invention is MK-7 or MK-4. Vitamin K2 is formulated to be administered at a daily dosage comprised between 5 to 100 µg per day, preferably 10 to 50 µg per day, more preferably around 20 µg per day (i.e. between 18 and 22 µg per day). This amount may be administered in a single dose or in more than one dose which may be taken at different times throughout the day.

The skilled person knows how to formulate vitamin K2 for administration in a patient in the context of the present invention. Vitamin K2 may be administered orally and be formulated in a solid form such as a tablet, capsule or chewable tablet or may be in a liquid form such as a solution, suspension, dispersion or syrup. Soft-gel capsules, in which the active ingredients are dissolved or dispersed in a liquid non-aqueous solution, are particularly suitable for administering vitamin K in the context of the present invention. Soft gel capsules may be prepared by dissolving or suspending vitamin K2 and any excipients or other desirable formulation aids in an oily medium which is then encapsulated in the soft gel capsule. Alternatively, vitamin K2 may also be formulated for transdermal administration, e.g. in the form of a cream or gel, or of a patch which may be adhesively attached to the skin and contains a reservoir of the vitamin K2 optionally in combination with a penetration enhancer or other suitable excipients. Vitamin K2 can also be administered intravenously, and formulated as a solution suitable for i.v. administration. Finally, it is also possible to deliver or administer vitamin K2 in nutritional products such as a fortified food or beverage product. Preferred nutritional product formats include: juice drinks, dairy drinks, powdered drinks, sports drinks, mineral water, soy beverages, hot chocolate, malt drinks, biscuits, bread, crackers, confectioneries, chocolate, chewing-gum, margarines, spreads, yogurts, breakfast cereals, snack bars, meal replacements, protein powders, desserts, and medical nutrition tube feeds and nutritional supplements.

These dosage forms may be prepared by methods which are well-known to those skilled in the art.

Conventional additives, but are not limited, may be included in the kit of the invention, including any of those selected from preservatives, chelating agents, effervescing agents, natural or artificial sweeteners, flavoring agents, coloring agents, taste masking agents, acidulants, emulsifiers, thickening agents, suspending agents, dispersing or wetting agents, antioxidants, and the like.

The present invention will now be illustrated by means of the following examples.

### Examples

### Introduction

Osteoporosis is a worldwide problem affecting more than 200 million people. In this invention, a combination of yeast and vitamin K2 was found to result in the production of beneficial bone health markers (i.e. metabolites measured during an *in vitro* human fermentation study). The yeast probiotics corresponding to the *S*. *Cerevisiae* strain deposited on October 17, 2007, at the Collection Nationale de Cultures de Micro-organismes (CNCM) pursuant to the treaty of Budapest under No. CNCM I-3856 in combination with Vitamin K2 menaquinone-7 (VitaMK-7^{®} - Gnosis by Lesaffre, FRANCE) showed beneficial combined effects in an *in vitro* human batch fermentation model of 48 hours, in terms of metabolite profile. Specific markers known to promote bone health were significantly increased with the combo during colonic fermentation as compared to CNCM I-3856 or Vitamin K2 alone. To our surprise the combo maintains or improves the positive bone markers associated to probiotic intake while it reduces or erase the negative markers observed with CNCM I-3856 alone. Altogether the results provided herein show a strong impact of CNCM I-3856 on positive bone markers, and a biological synergy of the combo to promote bone health.

### Materials and methods

The SHIME (Simulator of the Human Intestinal Microbioal Ecosystem, ProDigest) model was used in a batch fermentation set-up, where 200mL of sugar-depleted growth medium (1.2 g/LArabinogalactan, 2 g/L Pectin, 0.5 g/L xylan, 0.4 g/L glucose, 3 g/L yeast extract, 1 g/L special pepton, 3 g/L mucin and 0.5 g/L L-cystein-HCl) was supplemented with the combination of CNCM I-3856 (the strain strain deposited on October 17, 2007, at the Collection Nationale de Cultures de Micro-organismes (CNCM) pursuant to the treaty of Budapest under No. CNCM I-3856) (1g/intake) and Vitamin K2 MK7 Menaquinone (10mg/intake) and inoculated with a 10% fecal slurry of 2 individual donors (experiment repeated twice).

The reactors were individually and daily flushed with nitrogen to maintain anaerobic conditions. Samples were collected at T0, T24h and T48h and the short-chain fatty acid (SCFA), ammonium, lactate and branched-chain fatty acids (BCFA) were measured, as well as the microbial composition (16S metabarcoding) after 48h of fermentation. On a selection of samples, bone health biomarkers related to tryptophan metabolism were quantified using a targeted metabolomics approach.

### Results

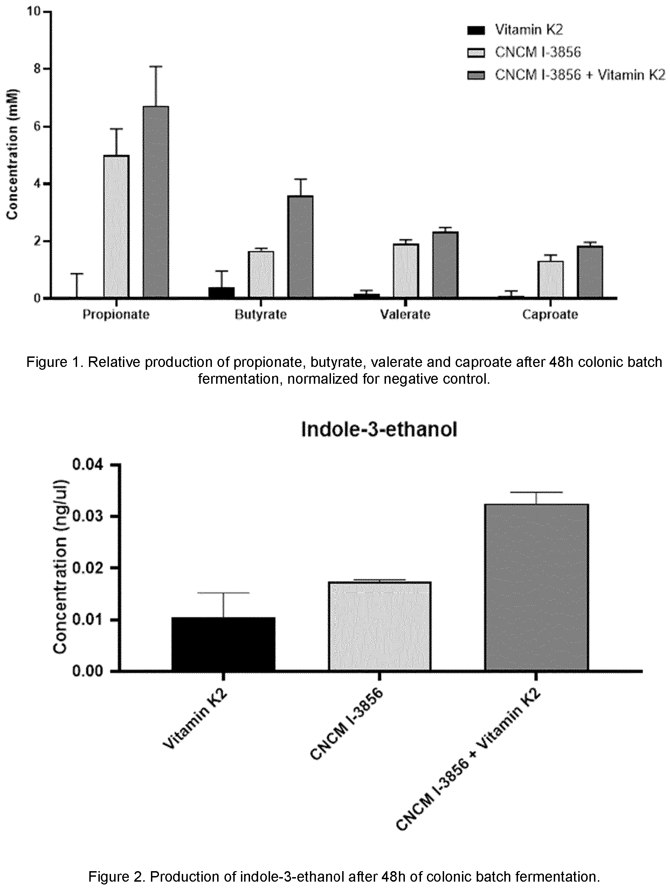

Results show an increase in the SCFAs, especially butyrate and propionate, when CNCM I-3856 was combined with Vitamin K2, compared to both products similarly dosed as single supplements (Figure 1). Propionate is known to trigger bone formation through osteocalcin gene expression modulation.

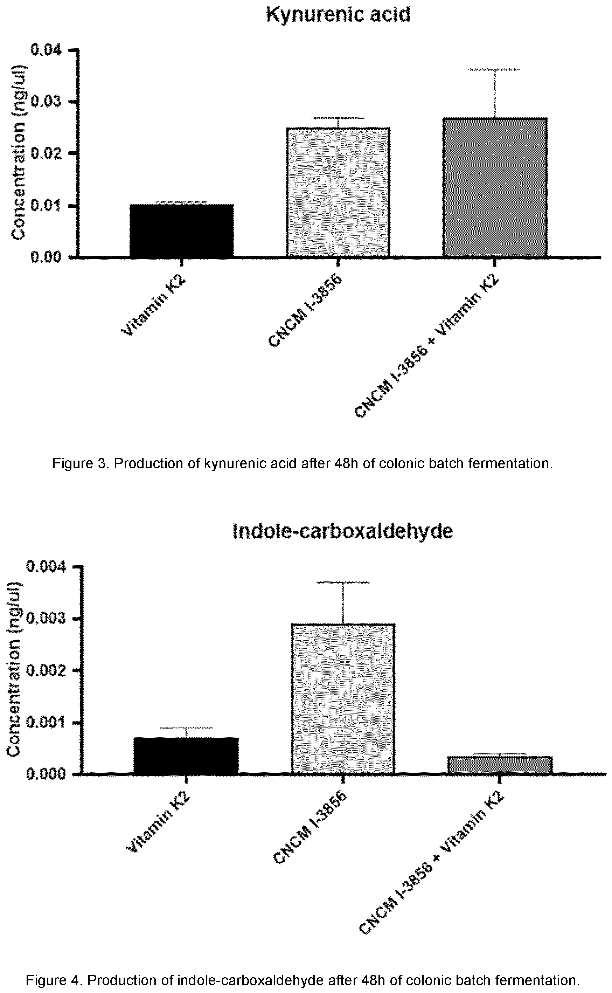

The metabolomics analysis conducted in those biological samples after human fermentation under additional Vitamin K2 and CNCM I-3856 showed that anti-inflammatory marker indole-3-ethanol was increased when the combo was supplemented compared to the Vitamin K2 and CNCM I-3856 alone (Figure 2). Besides, kynurenic acid, a biomarker known for bone formation and remodeling was also increased when comparing the combo with Vitamin K2 alone (Figure 3). On the other hand, the pro-osteoclastogenic biomarker indole-carboxaldehyde was decreased when the combo was supplemented compared to the supplementation of CNCM I-3856 alone, showing a corrective beneficial effect unexpected from the combo (Figure 4).

### Conclusion

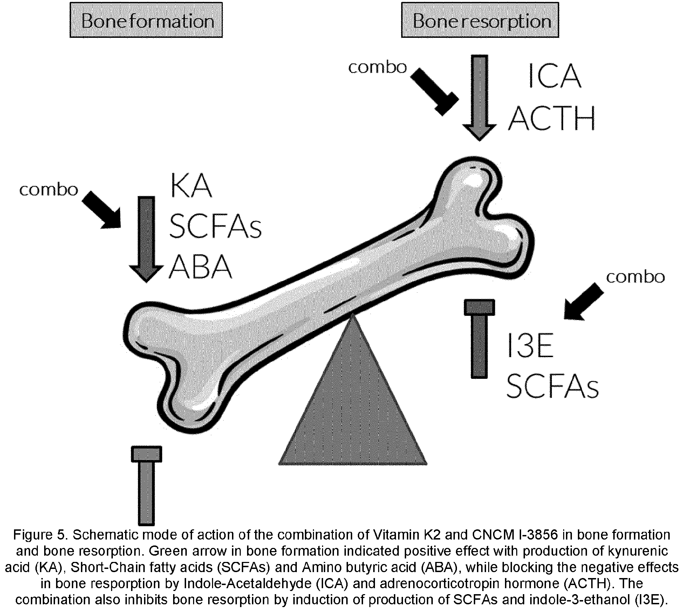

In conclusion this invention has shown that the use of CNCM I-3856 alone allows strongly promoting several markers involved in bone formation. The results further shown that supplementation of the combo Vitamin K2 and CNCM I-3856 synergistically interact so as to positively promote several markers involved in bone formation while mitigating expression of several markers of bone resorption (Figure 5). The supplementation of CNCM I-3856 alone, and more importantly of the combination CNCM I-3856 and Vitamin K2, can therefore result in a net positive role in bone health and prevention of osteoporosis.

## Claims

1. A live *Saccharomyces cerevisiae* strain for use in the treatment and/or prevention of a bone-loss disorder in a patient in need thereof.

2. The live *Saccharomyces cerevisiae* strain for use according to claim 1, wherein said *Saccharomyces cerevisiae* strain is not administered in combination with a microorganism that does not belong to the *Saccharomyces cerevisiae* species.

3. A kit of parts comprising:
- a live *Saccharomyces cerevisiae* strain; and
- vitamin K2.

4. The kit of parts according to claim 3, for use in therapy.

5. The kit of parts according to claim 3, for use in the treatment and/or prevention of a bone-loss disorder in a patient in need thereof.

6. The live *Saccharomyces cerevisiae* strain for use according to claim 1 or 2, or the kit of parts according to any one of claims 3 to 5, wherein said live *Saccharomyces cerevisiae* strain comprises the strain deposited on October 17, 2007, at the Collection Nationale de Cultures de Micro-organismes (CNCM) pursuant to the treaty of Budapest under No. CNCM I-3856.

7. The live *Saccharomyces cerevisiae* strain for use according to claim 1 or 2, or the kit of parts for use according to any one of claims 4 to 6, wherein said strain is administered to the patient at a daily dosage of between 10⁷ and 6×10¹⁰ CFU (Colony Forming Unit) of *Saccharomyces cerevisiae,* preferably between 10⁸ and 2×10¹⁰ CFU.

8. The live *Saccharomyces cerevisiae* strain for use according to claim 1 or 2, or the kit of parts for use according to any one of claims 4 to 7, wherein said bone-loss disorder is selected from the group consisting of osteoporosis, osteopenia, osteoarthritis, Paget's disease, osteomyelitis, osteomalacia, degenerative joint disease, musculoskeletal pathologies, osteophytes, rickets and delayed or non-union fractures.

9. The live *Saccharomyces cerevisiae* strain for use according to claim 1 or 2, or the kit of parts for use according to any one of claims 4 to 8, wherein said bone-loss disorder is selected from the group consisting of osteoporosis and osteopenia.

10. The live *Saccharomyces cerevisiae* strain for use according to claim 1 or 2, or the kit of parts for use according to any one of claims 4 to 9, wherein said bone disorder is osteoporosis.

11. The kit of parts for use according to any one of claims 4 to 10, wherein vitamin K2 is administered at a daily dosage comprised between 5 to 100µg per day, preferably 10 to 50 µg per day.

12. The kit of parts for use according to any one of claims 4 to 11, wherein said vitamin K2 is MK-7 and/or MK-4.

13. The kit of parts for use according to any one of claims 4 to 12, wherein the live *Saccharomyces cerevisiae* strain and vitamin K2 are administered simultaneously.

14. The kit of parts for use according to any one of claims 4 to 12, wherein the live *Saccharomyces cerevisiae* strain and vitamin K2 are administered subsequentially.
